# EUROPÄISCHE PATENTSCHRIFT

(11) **EP 1 509 228 B1**
(45) Veröffentlichungstag und Bekanntmachung des Hinweises auf die Patenterteilung: **14.12.2005**
(21) Anmeldenummer: 03722593.5
(22) Anmeldetag: 05.05.2003
(51) Int. Cl.: A61K 31/505, A61P 7/02, A61P 9/00, A61P 9/10, A61P 9/12, A61P 15/10, C07D 471/04

(54) **DERIVATE-1H-PYRAZOLO (3, 4-B) -5-(4-PYRIDINYL)-4-PYRIMIDINAMINS UND IHRE VERWENDUNG ALS GUANYLATCYCLASE-STIMULATOREN**
DERIVATIVES OF 2-(1-BENZYL-1H-PYRAZOLO (3, 4-B)PYRIDINE-3YL) -5-(4-PYRIDINYL)-4-PYRIMIDINE AMINE AND THE USE THEREOF AS GUANYLATE CYCLASE STIMULATORS
DERIVES DE LA 2-(1-BENZYL-1H-PYRAZOLO (3,4-B)PYRIDINE-3-YL)-5-(4-PYRIDINYL)-4-PYRIMIDINAMINE ET LEUR UTILISATION EN TANT QU'AGENTS DE STIMULATION DE LA GUANYLATE CYCLASE

(30) Priorität: 17.05.2002 DE 10222550
(43) Veröffentlichungstag der Anmeldung: 02.03.2005
(73) Patentinhaber: Bayer HealthCare AG, 51368 Leverkusen (DE)
(72) Erfinder: WEIGAND, Stefan, 42115 Wuppertal (DE); BISCHOFF, Erwin, 42115 Wuppertal (DE); MÜNTER, Klaus, 42489 Wülfrath (DE); STASCH, Johannes-Peter, 42651 Solingen (DE); STAHL, Elke, 51467 Bergisch Gladbach (DE)
(86) Internationale Anmeldenummer: PCT/EP2003/004668
(87) Internationale Veröffentlichungsnummer: WO 2003/097063

(56) Entgegenhaltungen:
- WO-A-00/06567
- WO-A-00/06568
- WO-A-00/06569
- WO-A-02/42301

## Beschreibung

Die vorliegende Erfindung betrifft chemische Verbindungen, die die lösliche Guanylatcyclase stimulieren, ihre Herstellung und ihre Verwendung als Arzneimittel, insbesondere als Arzneimittel zur Behandlung von Herz-Kreislauf-Erkrankungen und/oder sexueller Dysfunktion.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodinner, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall, sexueller Dysfunktion und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in WO 98/16507, WO 98/23619, WO 00/06567, WO 00/06568, WO 00/06569, WO 00/21954 WO 02/42299, WO 02/42300, WO 02/42301, WO 02/42302, WO 02/092596 und WO 03/004503 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf. Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg einige Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindung durch die Verbindungen gemäß Anspruch 1 gelöst. Diese neuen Pyrazolopyridinderivate zeichnen sich durch einen 4-Amino-5-(Pyridin-4-yl)-pyrimidinrest in 3-Position sowie einen substituierten Benzylrest in 1-Position aus.

Im einzelnen betrifft die vorliegende Erfindung Verbindungen der Formel (I) 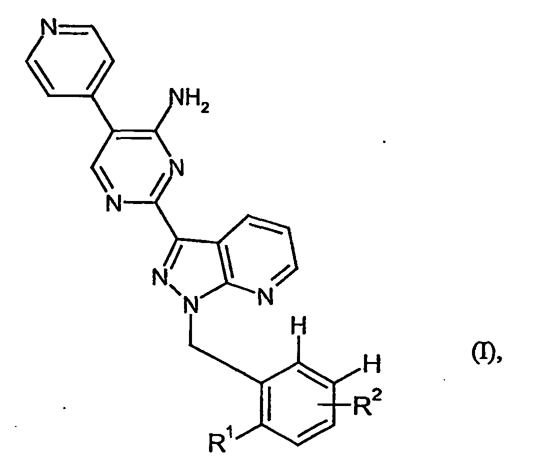 worin
- R¹: für Chlor, Cyano, Trifluormethyl oder Methoxy steht
und
- R²: für Wasserstoff oder Fluor steht
oder
- R¹: für Fluor steht und
- R²: für Fluor steht,
sowie Salze, Isomere und Hydrate davon.

Die erfindungsgemäßen Verbindungen der Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindung können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindung sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in tautomeren Formen vorliegen. Dies ist dem Fachmann bekannt, und derartige Formen sind ebenfalls vom Umfang der Erfindung umfasst.

Weiterhin können die erfindungsgemäßen Verbindungen in Form ihrer möglichen Hydrate vorkommen.

Ein Symbol * an einer Bindung bedeutet die Verknüpfungsstelle im Molekül.

Bevorzugt sind Verbindungen der Formel (Ia) 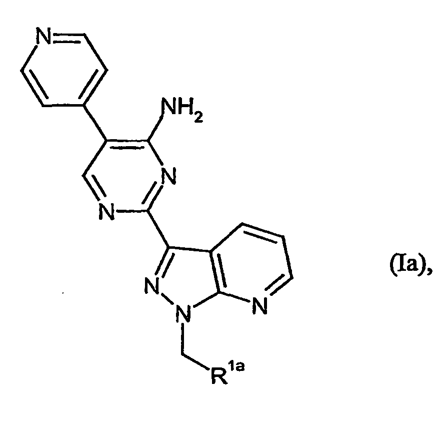 worin
- R^{1a}: ausgewählt ist aus der Gruppe von 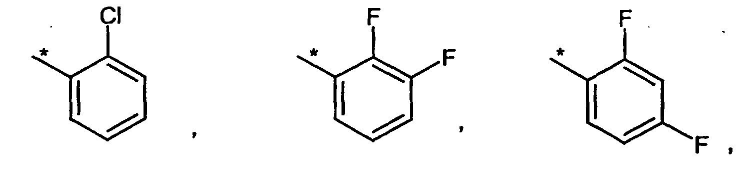 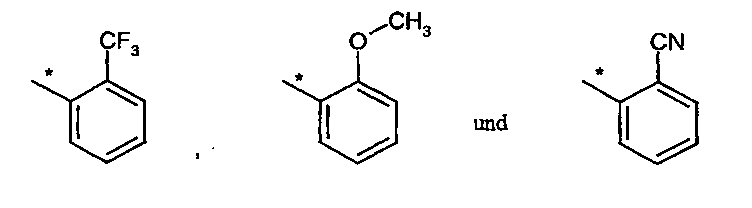
sowie Salze, Isomere und Hydrate davon.

Bevorzugt ist die Verbindung der Formel (Ia), bei der
- R^{1a}: für 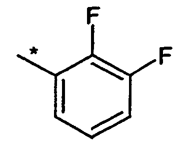
steht,
sowie Salze, Isomere und Hydrate davon.

Die Herstellung der erfindungsgemäßen Verbindungen der Formel (I) kann nach üblichen, dem Fachmann geläufigen Reaktionsschritten erfolgen, beispielsweise analog den bei der Synthese der Ausführungsbeispiele beschriebenen Verfahren.

Die erfindungsgemäßen Verbindungen der Formel (I) zeigt ein nicht vorhersehbares, wertvolles pharmakologisches Wirkspektrum.

Die erfindungsgemäßen Verbindungen der Formel (I) bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatzyklase und einen intrazellulären cGMP-Anstieg vermittelt. Außerdem verstärken die erfindungsgemäßen Verbindungen der Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivaten.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese und Inkontinenz eingesetzt werden.

Die erfindungsgemäßen Verbindungen der Formel (I) sind auch zur Bekämpfung von Krankheiten im Zentralnervensystem geeignet, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimerscher Krankheit, Demenz mit Lewy-Körperchen, Demenz mit Degeneration der Frontallappen einschliesslich des Pick's Syndroms, Parkinsonscher Krankheit, progressiver nuclear palsy, Demenz mit corticobasaler Degeneration, Amyolateralsklerose (ALS), Huntingtonscher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die erfindungsgemäßen Verbindungen der Formel (I) auch zur Regulation der cerebralen Durchblutung und stellen somit wirkungsvolle Mittel zur Bekämpfung von Migräne dar.

Auch eignen sich die erfindungsgemäßen Verbindungen der Formel (I) zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können sie zur Bekämpfung von Schmerzzuständen eingesetzt werden.

Zudem besitzen die erfindungsgemäßen Verbindungen der Formel (I) antiinflammatorische Wirkung und können daher als entzündungshemmende Mittel eingesetzt werden.

Darüber hinaus umfasst die vorliegende Erfindung auch die Kombination mindestens einer erfindungsgemäßen Verbindungen der Formel (I) mit einem oder mehreren organischen Nitraten oder NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die vorliegende Erfindung auch die Kombination mit einer oder mehreren Verbindungen, die den Abbau von cyclischem Guanosinmonophosphat (cGMP) inhibieren. Dies sind vorzugsweise Inhibitoren der Phosphodiesterasen 1, 2 und 5; Nomenklatur nach Beavo und Reifsnyder (1990) TiPS 11 S. 150 bis 155. Besonders bevorzugt sind hierbei Inhibitoren der Phosphodiesterase 5 (PDE V-Hemmer), insbesondere eine der Verbindungen Sildenafil (Viagra™, EP-A 0 463 756, WO 94/28902), Vardenafil (WO 99/24433) oder Tadalafil (WO 95/19978). Durch diese Inhibitoren wird die Wirkung der erfindungsgemäßen Verbindungen potenziert und der gewünschte pharmakologische Effekt gesteigert.

Weiterer Gegenstand der vorliegenden Erfindung sind Arzneimittel, die mindestens eine erfindungsgemäße Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, topisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate wie beispielsweise Stents.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien. Der Wirkstoff kann gegebenenfalls in einem oder mehreren der oben angegebenen Trägerstoffe auch in mikroverkapselter Form vorliegen.

Die therapeutisch wirksame Verbindung der Formel (I) soll in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer der erfindungsgemäßen Verbindung der Formel (I) auch weitere pharmazeutische Wirkstoffe enthalten.

Im allgemeinen hat es sich sowohl in der Human- als auch in der Veterinärmedizin als vorteilhaft erwiesen, den erfindungsgemäßen Wirkstoff in Gesamtmengen von etwa 0,001 bis etwa 50, vorzugsweise 0,001 bis 10 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff vorzugsweise in Mengen von etwa 0,001 bis etwa 30, insbesondere 0,001 bis 3 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente. Lösungsmittelverhältnisse, Verdünnungsverhältnisse und Konzentrationsangaben von flüssig/flüssig-Lösungen beziehen sich jeweils auf das Volumen.

### Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogen-begaster Krebs-Henseleit-Lösung folgender Zusammensetzung (mM) gebracht: NaCl: 119; KCI: 4,8; CaCl₂ x 2 H₂O: 1; MgSO₄ x 7 H₂O: 1,4; KH₂PO₄: 1,2; NaHCO₃: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl, der DMSO-Anteil in der Badlösung entspricht 0,1 %.

### Kaninchen-Modell

Adulte, männliche Chinchilla-Kaninchen mit einem Gewicht von 3 - 5 kg werden nach Lieferung mehrere Tage in Einzelhaltung adaptiert. Sie haben freien Zugang zu Wasser und können zwei Stunden pro Tag Futter zu sich nehmen. Die Tiere werden in einem 10/14 Stunden Tag-Nacht Rhythmus gehalten (Licht an, ab 8.00 Uhr), die Raumtemperatur beträgt 22 - 24°C.

Pro Behandlungsgruppe werden drei bis sechs Tiere verwendet und direkt vor Versuchsbeginn gewogen. Für die i.v. Gabe werden die Substanzen in Transcutol (GATTEFOSSE GmbH) gelöst und im Verhältnis 3/7 mit einer 20%igen Cremophorlösung (Cremophor (BASF), Wasser) verdünnt. Es wird ein Volumen von 0,5 ml/kg in die Ohrvene injiziert. Wasserlösliche Substanzen werden in 0,9 % Kochsalzlösung injiziert.

Für die orale Gabe werden die Testsubstanzen in einer Mischung von Glycerin: Wasser: Polyethylenglycol 6:10:9.69 gelöst und in einem Volumen von 1 ml/kg mit der Schlundsonde appliziert.

Unter Ruhebedingungen ist der Kaninchenpenis in der Schamregion nicht sichtbar und von der Penishaut vollständig bedeckt. Die Erektion wird gewertet, indem man die Länge des hervortretenden Penis mit einer Schiebelehre misst. Die Messung wird 5, 10, 15, 30, 45, 60 und120 Minuten nach Substanzgabe durchgeführt, nach oraler Gabe zusätzlich auch noch nach 3, 4, 5 und 6 Stunden. Die Tiere werden dazu jedes Mal aus dem Käfig geholt, am Nackenfell und den Hinterläufen festgehalten, auf den Rücken gedreht und gemessen. Entsprechende Lösungsmittelkontrollen werden durchgeführt. (vergleiche Literatur: E. Bischoff, K. Schneider, Int. J. of Impotence Res. 2001, 13, 230-235; E. Bischoff, U. Niewoehner, H. Haning, M. Es Sayed, T. Schenke, K. H. Schlemmer, The Journal of Urology, 2001, 165, 1316-1318; E. Bischoff, Int. J. Impotence Res. 2001, 13, 146-148).

### Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen, dieses wird heparinisiert und anschließend wird daraus durch Zentrifugation Plasma gewonnen. Die Substanz wird im Plasma über LC/MSMS analytisch quantifiziert. Aus den so ermittelten Plasmakonzentrations-Zeit-Verläufen werden die pharmakokinetischen Kenngrößen mittels eines validierten pharmakokinetischen Rechenprogramms berechnet.

### Inhibition von Cytochrom P450-Enzymen

Das Potential der Inhibition von P-450 Isoenzymen, die für den Metabolismus wichtig sind, wird automatisiert im 96-well Format untersucht. Hierbei werden zwei verschiedene Assays verwendet.

Bei dem auf Bildung von fluoreszierenden Metaboliten basierenden. Assay werden rekombinante Enzyme (z.B. CYP1A2, 2C8, 2C9, 2C19, 2D6 oder 3A4) und im allgemeinen Fluorescein- oder Coumarin-Teilstrukturen enthaltene Substrate eingesetzt. Es werden jeweils eine Substratkonzentration und 8 Konzentrationen des potentiellen Inhibitors verwendet. Nach Inkubation mit dem jeweiligen rekombinanten CYP Enzym wird mittels Fluoreszenzreader das Ausmaß an fluoreszierenden Metaboliten im Vergleich zur Kontrolle (ohne Inhibitor) ermittelt und ein IC₅₀-Wert berechnet [Anal. Biochem. **248**, 188 (1997)].

Beim 2. Assay werden als Enzymquelle humane Lebermikrosomen und als CYP Isoform-selektive Substrate Phenacetin (CYP1A2), Diclofenac (CYP2C9), Dextromethorphan (CYP2D6) und Midazolam (CYP3A4) verwendet. Die Bildung des jeweiligen Metaboliten wird mittels LC-MS/MS gemessen. Unter Annahme kompetitiver Inhibition werden aus der Verminderung der Metabolitenbildung im Vergleich zur Kontrolle Kᵢ-Werte berechnet (1 Substrat-, 3 Inhibitorkonzentrationen).

### Induktion von Cytochrom P450-Enzymen in humanen Leberzellkulturen

Zur Untersuchung des Nebenwirkungspotentials der erfindungsgemäßen Substanzen bezüglich einer Induktion von Cytochrom P450-Enzymen werden primäre humane Hepatozyten mit einer Zelldichte von 2,5 x 10⁵ Zellen zwischen zwei Schichten von Collagen in 24 well-Mikrotiterplatten bei 37°C bei 5 % CO₂ 8 Tage kultiviert. Das Zellkulturmedium wird täglich gewechselt.

Nach 48 Stunden in Kultur werden die Hepatozyten über 5 Tage in Doppelbestimmung mit unterschiedlichen Konzentrationen der Testsubstanzen im Vergleich mit den Induktoren Rifampicin (RIF; 50 µM), Omeprazol (OME; 100 µM) und Phenobarbital (PB; 2 mM) behandelt. Die Endkonzentrationen der Testsubstanzen liegen bei 0,01 - 10 µg/ml.

Von den Zellkulturen wird der induktive Effekt der Testsubstanzen auf die Cytochrom (CYP) P450-Enzyme 1A2, 2B6, 2C19 und 3A4 durch Zugabe der Substrate 7-Ethoxyresorufin (CYP1A2), [¹⁴C]-S-Mephenytoin (CYP2B6 und 2C19) und [¹⁴C]-Testosteron (CYP3A4) am Tag 8 bestimmt. Von den so gemessenen Enzymaktivitäten CYP1A2, 2B6, 2C19 und 3A4 behandelter Zellen im Vergleich zu unbehandelten Zellen wird das induktive Potential der Testsubstanzen ermittelt.

### Synthese von Ausgangsverbindungen und Ausführungsbeispielen:

### Abkürzungen:

- ACN: Acetonitril
- Äquiv.: Äquivalent
- DC: Dünnschichtchromatographie
- DCI: direkte chemische Ionisation (bei MS)
- DCM: Dichlormethan
- DIEA: *N,N*-Diisopropylethylamin
- DMAP: Dimethylaminopyridin
- DMSO: Dimethylsulfoxid
- DMF: *N,N*-Dimethylformamid
- d. Th.: der Theorie
- EE: Ethylacetat (Essigsäureethylester)
- EI: Elektronenstoß-Ionisation (bei MS)
- eq.: Äquivalent
- ESI: Elektrospray-Ionisation (bei MS)
- Fp.: Schmelzpunkt
- ges.: gesättigt
- H: Stunde
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- MS: Massenspektroskopie
- NMR: Kernresonanzspektroskopie
- RP-HPLC: Reverse Phase HPLC
- RT: Raumtemperatur
- Rₜ: Retentionszeit (bei HPLC)
- THF: Tetrahydrofuran

### LC/MS und HPLC-Methoden:

### Methode 1 (LCMS)

Instrument: Micromass Platform LCZ, HP1100; Säule: Symmetry C18, 50 mm x 2,1 mm, 3,5 µm; Eluent A: Wasser + 0,05 % Ameisensäure, Eluent B: Acetonitril + 0,05 % Ameisensäure; Gradient: 0,0 min 90 % A -> 4,0 min 10 % A -> 6,0 min 10 % A; Ofen: 40°C; Fluss: 0,5 ml/min; UV-Detektion: 208-400 nm.

### Methode 2 (LCMS)

Instrument: Waters Alliance 2790 LC; Säule: Symmetry C18, 50 mm x 2,1, 3,5 µm; Eluent A: Wasser + 0,1 % Ameisensäure, Eluent B: Acetonitril + 0,1 % Ameisensäure; Gradient: 0,0 min 5 % B → 5,0 min 10 %B → 6,0 min 10 % B; Temperatur: 50°C; Fluss: 1,0ml/min; UV-Detektion: 210 nm.

### Methode 3 (HPLC)

Instrument: HP 1100 mit DAD-Detektion; Säule: Kromasil RP-18, 60 mm x 2 mm, 3,5 µm; Eluent: A=5 ml HClO₄/l H₂O, B=ACN; Gradient: 0 min 2 % B, 0,5 min 2 % B, 4,5 min 90 % B, 6,5 min 90 %B; Fluß: 0,75 ml/min; Temp.: 30°C; Detektion UV 210 nm.

### Präparative RP-HPLC

Säule: YMC-Gel; Eluent: Acetonitril/Wasser (Gradient); Fluß: 50 ml/min; Temp.: 25°C; Detektion UV 210 nm.

### Ausgangsverbindungen:

### Beispiel 1 A

### 1-(2-Chlorbenzyl)hydrazin

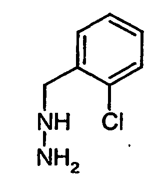

2,74 g (54,75 mmol) Hydrazinhydrat werden in 10 ml Methanol vorgelegt und mit einer Lösung von 3,00 g (14,60 mmol) 2-Chlorbenzylbromid in 5 ml Methanol bei RT versetzt. Dabei steigt die Temperatur auf 35-40°C, die Mischung wird 3 Stunden bei RT nachgerührt. Es wird im Vakuum das Lösungsmittel entfernt und der Rückstand in 100 ml Diethylether aufgenommen, über Magnesiumsulfat getrocknet und abfiltriert.
Gesamtausbeute: 2,34 g (100 % d. Th.)
LC/MS (Methode 2): Rₜ = 0,37 min
MS (EI): m/z = 157 (M+H)⁺
¹H-NMR (200 MHz, DMSO-d₆): δ = 3,29-3,59 (s, 2H), 3,84 (s, 2H), 7,18-7,56 (m, 4H), 10,22 (br. s, 1H).

### Beispiel 2 A

### 1-(2,3-Difluorbenzyl)hydrazin

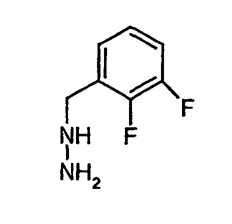

Die Herstellung erfolgt analog zu der in Beispiel 1 A beschriebenen aus 2,74 g (54,75 mmol) Hydrazinhydrat und 3,02 g (14,60 mmol) 2,3-Difluorbenzylbromid. Zur Aufarbeitung wird der Rückstand flash-chromatographisch (Laufmittel: Dichlormethan:Methanol 30:1-10:1) gereinigt.
Gesamtausbeute: 1,51 g (65 % d. Th.)
LC/MS (Methode 2): Rₜ = 0,32 min
MS (EI): m/z = 159 (M+H)⁺
¹H-NMR (200 MHz, DMSO-d₆): δ = 3,75-3,88 (m, 2H), 4,61-4,94 (br. s, 3H), 7,07-7,39 (m, 3H).

Die Herstellung der folgenden Verbindungen erfolgt analog zu der in Beispiel 1 A beschriebenen:

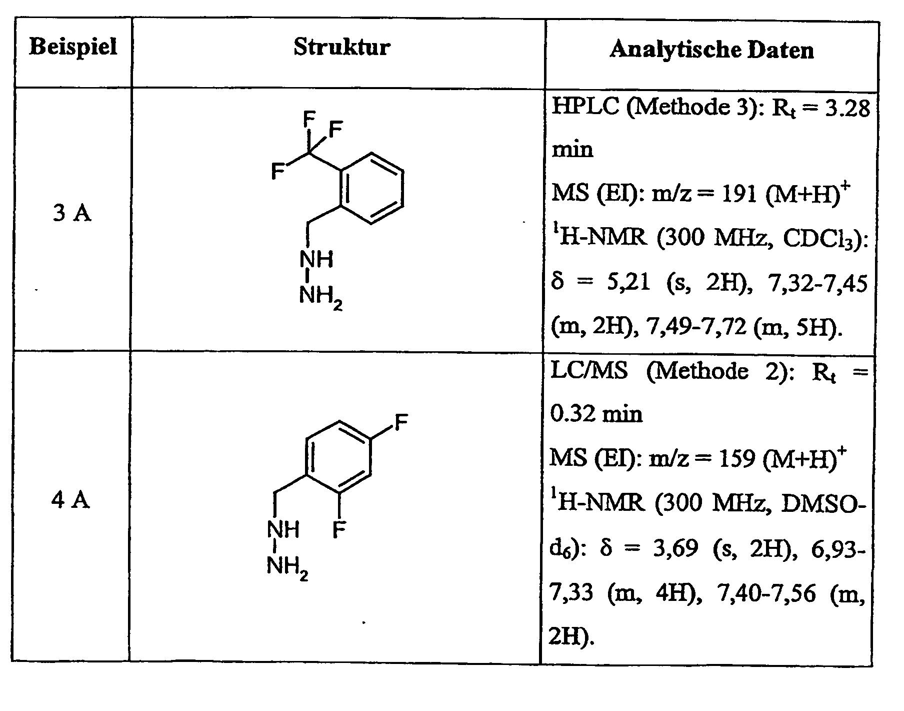

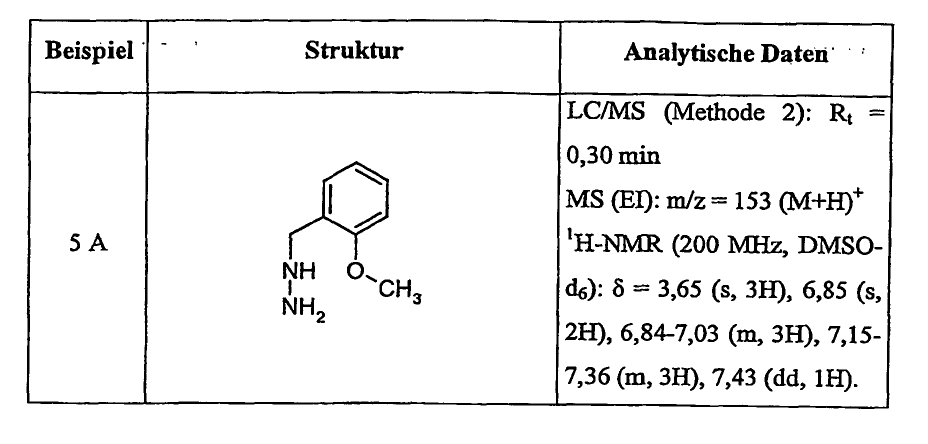

### Beispiel 6 A

### Natrium (1E)-1-cyano-3-ethoxy-3-oxo-1-propen-2-olat

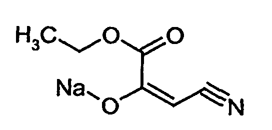

517 g (7,60 mol) Natriumethylat werden in 3000 ml Diethylether vorgelegt und unter Eiskühlung mit 1121 g (7,60 mol) Oxalsäurediethylester innerhalb von 35 Minuten versetzt. Man rührt 15 Minuten nach und kühlt wieder ab. Innerhalb von 20 Minuten wird 312 g (7,60 mol) Acetonitril zugetropft. Es wird über Nacht bei RT gerührt und die entstandenen Kristalle werden abgesaugt, mit Diethylether gewaschen und getrocknet. Gesamtausbeute: 1030 g (83 % d. Th.)
¹H-NMR (300 MHz, CDCl₃): δ = 1,27 (t, 3H), 4,17 (q, 2H), 7,60 (s, 1H).

### Beispiel 7 A

### 5-Amino-1-(2-chlorbenzyl)-1H-pyrazol-3-carbonsäureethylester

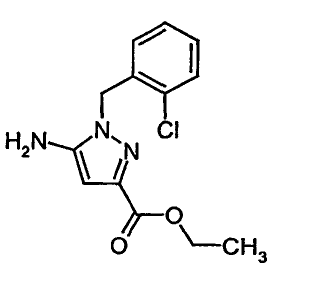

Unter Argon werden 2,29 g (14,60 mmol) 1-(2-Chlorbenzyl)hydrazin aus Beispiel 1 A in 60 ml Dioxan gelöst. Dazu gibt man 2,38 g 814,60 mmol) Natrium (1E)-1-cyano-3-ethoxy-3-oxo-1-propen-2-olat aus Beispiel 6 A und 2,66 g (1,80 ml; 23,36 mmol) Trifluoressigsäure. Das Gemisch wird über Nacht bei Rückfluss gekocht und ohne weitere Aufarbeitung weiter umgesetzt.
LC/MS (Methode 2): Rₜ = 2.45 min
MS (EI): m/z = 280 (M+H)⁺

### Beispiel 8 A

### 5-Amino-1-(2,3-difluorobenzyl)-1H-pyrazol-3-carbonsäureethylester

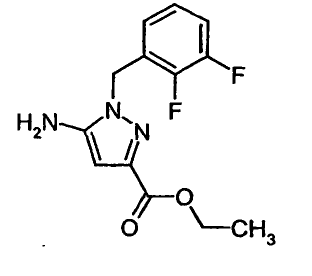

Die Herstellung erfolgt analog zu der in Beispiel 7 A beschriebenen aus 1,50 g (9,48 mmol) 1-(2,3-Difluorbenzyl)hydrazin aus Beispiel 2 A, 1,55 g (9,48 mmol) Natrium (1E)-1-cyano-3-ethoxy-3-oxo-1-propen-2-olat aus Beispiel 6 A, 1,73 g (1,17 ml; 15,18 mmol) Trifluoressigsäure und 40 ml Dioxan.
LC/MS (Methode 1): Rₜ = 3.90 min
MS (EI): m/z = 282 (M+H)⁺
¹H-NMR (200 MHz, DMSO-d₆): δ = 1,24 (t, 3H), 4,18 (q, 2H), 4,19-4,46 (br. s, 2H), 5,32 (s, 2H), 5,76 (s, 1H), 6,59-6,72 (m, 1H), 7,07-7,24 (m, 1H), 7,27- 7,46 (m, 1H).

Die Herstellung der folgenden Verbindungen erfolgt analog zu der in Beispiel 7 A beschriebenen:

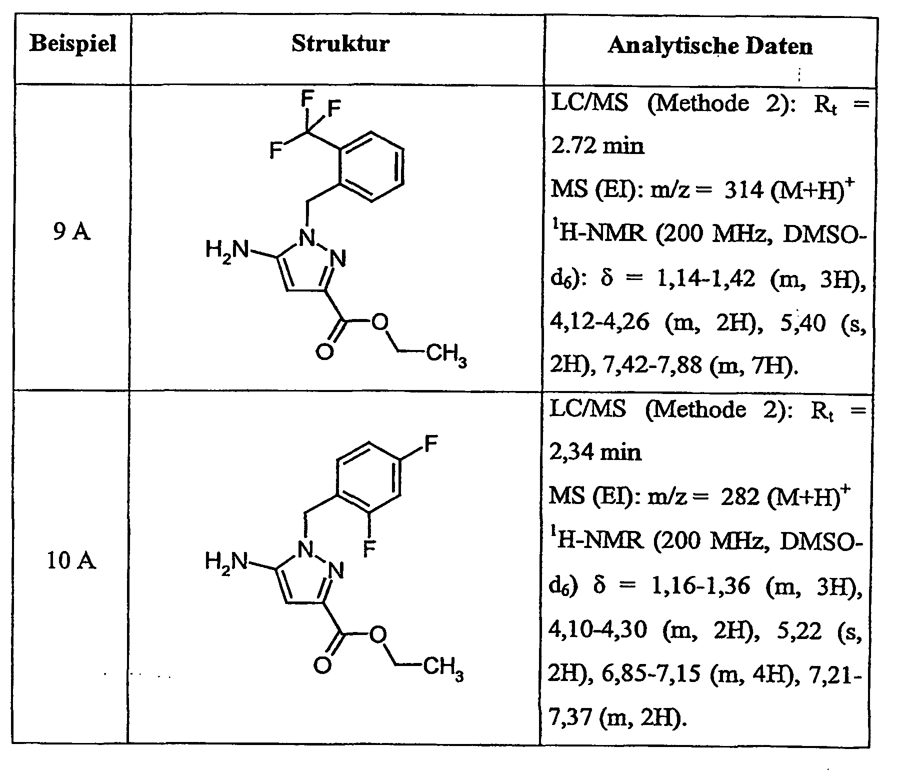

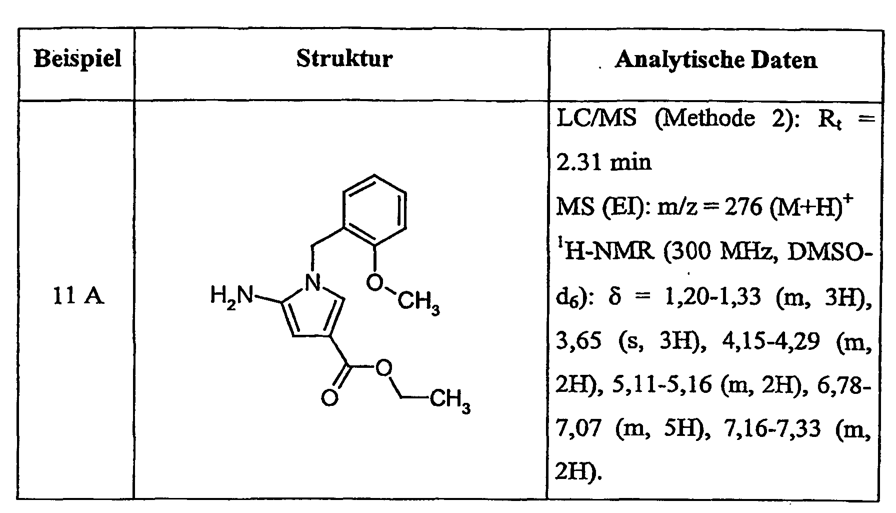

### Beispiel 12 A

### 1-(2-Chlorbenzyl)-1H-pyrazol[3,4-b]pyridin-3-carbonsäureethylester

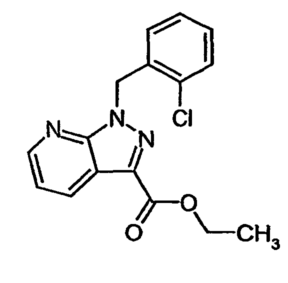

Unter Argon wird die Lösung von 4,08 g (14.60 mmol) 5-Amino-1-(2-chlorbenzyl)-1H-pyrazol-3-carbonsäureethylester aus Beispiel 7 A vorgelegt und mit 1,99 g (1,35 ml; 17,52 mmol) Trifluoressigsäure und 1,45 g (14,60 mmol) 3-Dimethylaminoacrolein versetzt. Es wird 3 Stunden zum Rückfluss gekocht und die Mischung zur Aufarbeitung im Vakuum vom Lösungsmittel befreit. Der Rückstand wird über Kieselgel (Laufmittel: Cyclohexan:Ethylacetat 7:1) flash-chromatographisch gereinigt.
Gesamtausbeute: 2,94 g (64 % d. Th.)
LC/MS (Methode 1): Rₜ = 4,74 min
MS (EI): m/z = 316 (M+H)⁺
¹H-NMR (300 MHz, DMSO-d₆): δ = 1,37 (t, 3H), 4,41 (q, 2H), 5,91 (s, 2H), 7,00-7,08 (m, 1H), 7,12-7,22 (m, 1H), 7,34-7,43 (m, 1H), 7,46-7,49 (m, 1H), 7,83 (d, 1H), 8,50 (dd, 1H), 8,71 (dd, 1H).

### Beispiel 13 A

### 1-(2,3-Difluorbenzyl)-1H-pyrazol[3,4-b]pyridin-3-carbonsäureethylester

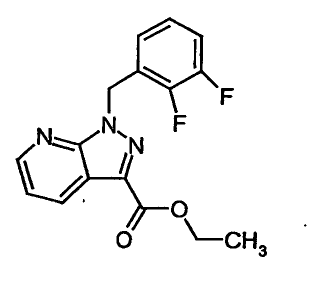

Die Herstellung erfogt analog zu der in Beispiel 12 A beschriebenen aus 4,11 g (14,60 mmol) 5-Amino-1-(2,3-difluorbenzyl)-1H-pyrazol-3-carbonsäureethylester aus Beispiel 8 A, 1,99 g (1,35 ml; 17,52 mmol) Trifluoressigsäure und 1,45 g (14,60 mmol) 3-Dimethylaminoacrolein.
Gesamtausbeute: 1,94 g (29 % d. Th.)
LC/MS (Methode 1): Rₜ = 3,31 min
MS (EI): m/z = 318 (M+H)⁺
¹H-NMR (300 MHz, DMSO-d₆): δ = 1.37 (t, 3H), 4,41 (q, 2H), 5,91 (s, 2H), 7,00-7,08 (m, 1H), 7,11-7,22 (m, 1H), 7,33-7,45 (m, 1H), 7,49 (dd, 1H), 8,50 (dd, 1H), 8,71 (dd, 1H).

Die Herstellung der folgenden Verbindungen erfolgt analog zu der in Beispiel 12 A beschriebenen:

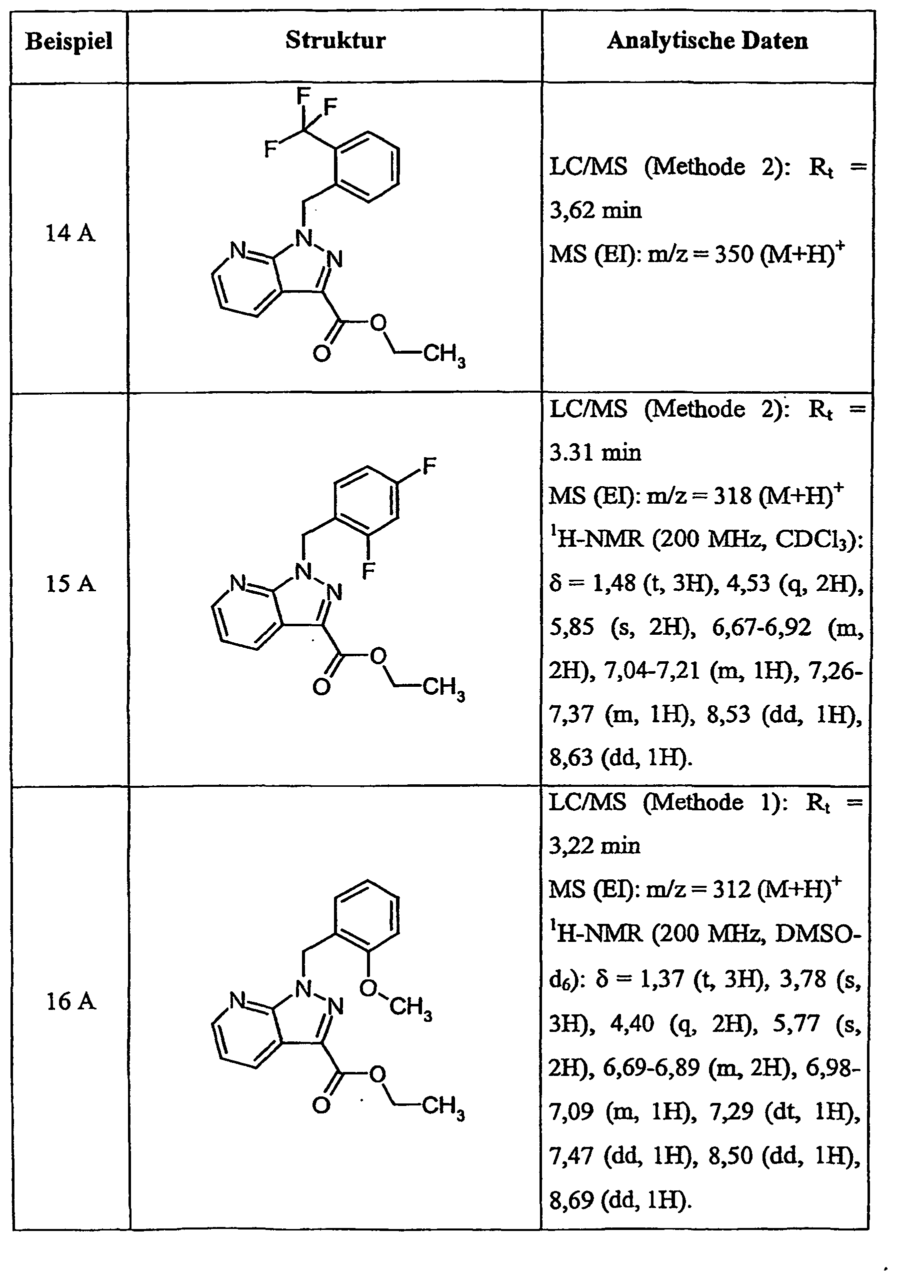

### Beispiel 17A

### 1-(2-Chlorbenzyl)-1H-pyrazol[3,4-b]pyridin-3-carboxamid

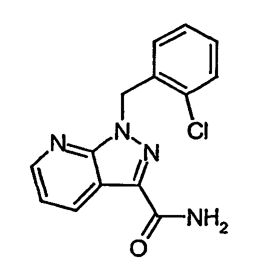

Bei Raumtemperatur werden 2,94 g (9,31 mmol) 1-(2-Chlorbenzyl)-1H-pyrazol[3,4-b]pyridin-3-carbonsäureethylester aus Beispiel 12 A in 50 ml 5,5 molarer Ammoniaklösung in Methanol suspendiert. Es wird für 16 Stunden bei RT gerührt und wieder zur Trockene einrotiert. Der Rückstand wird erneut mit 50 ml Ammoniaklösung versetzt und 3 Stunden bei 50°C gerührt. Das wird über 3 Tage wiederholt. Nach der letzten Trocknung wird der Rückstand in 40 ml Diethylether aufgenommen und die entstandenen Kristalle abgesaugt und getrocknet. Die Mutterlauge wird wieder einrotiert und die Mischung mit 50 ml Ammoniaklösung wieder versetzt und im Autoklaven bei 80°C im Eigendruck gerührt. Der Rückstand wird über Kieselgel (Laufmittel: Cyclohexan:Ethylacetat 5:1) flash-chromatographisch gereinigt. Gesamtausbeute: 1,33 g (50 % d. Th.)
LC/MS (Methode 1): Rₜ = 4,09 min
¹H-NMR (300 MHz, DMSO-d₆): δ = 5,85 (s, 2H), 6,87 (dd, 1H), 7,25 (dt, 1H), 7,34 (dt, 1H), 7,40 (dd, 1H), 7,51 (dd, 1H), 7,78 (br. s, 2H), 8,58 (dd, 1H), 8,64 (dd, 1H).

### Beispiel 18A

### 1-(2,3-Difluorbenzyl)-1H-pyrazol[3,4-b]pyridin-3-carboxamid

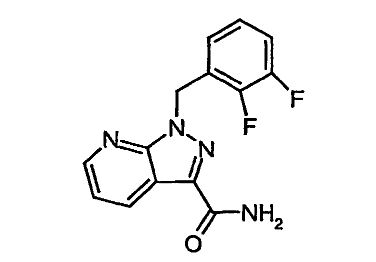

Bei Raumtemperatur werden 1,90 g (5.99 mmol) 1-(2,3-Difluorbenzyl)-1H-pyrazol[3,4-b]pyridin-3-carbonsäureethylester aus Beispiel 13 A in 50 ml 5,5 molarer Ammoniaklösung in Methanol suspendiert. Es wird für 16 Stunden bei RT gerührt und anschließend zur Trockene einrotiert. Es wird noch zwei mal mit Dichlormethan versetzt und zur Trockene einrotiert.
Gesamtausbeute: 0,87 g (50 % d. Th.)
LC/MS (Methode 1): Rₜ = 4,00 min
¹H-NMR (200 MHz, DMSO-d₆): δ = 5,86 (s, 2H), 6,90-7,03 (m, 1H), 7,07-7,22 (m, 1H), 7,29-7,49 (m, 2H), 7,71 (d, 2H), 8,57 (dd, 1H), 8,66 (dd, 1H).

Die Herstellung der folgenden Verbindungen erfolgt analog zu der in Beispiel 17 A beschriebenen:

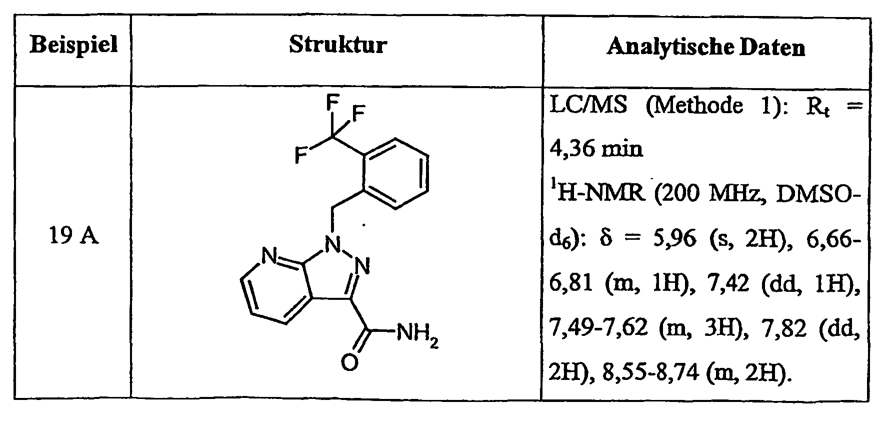

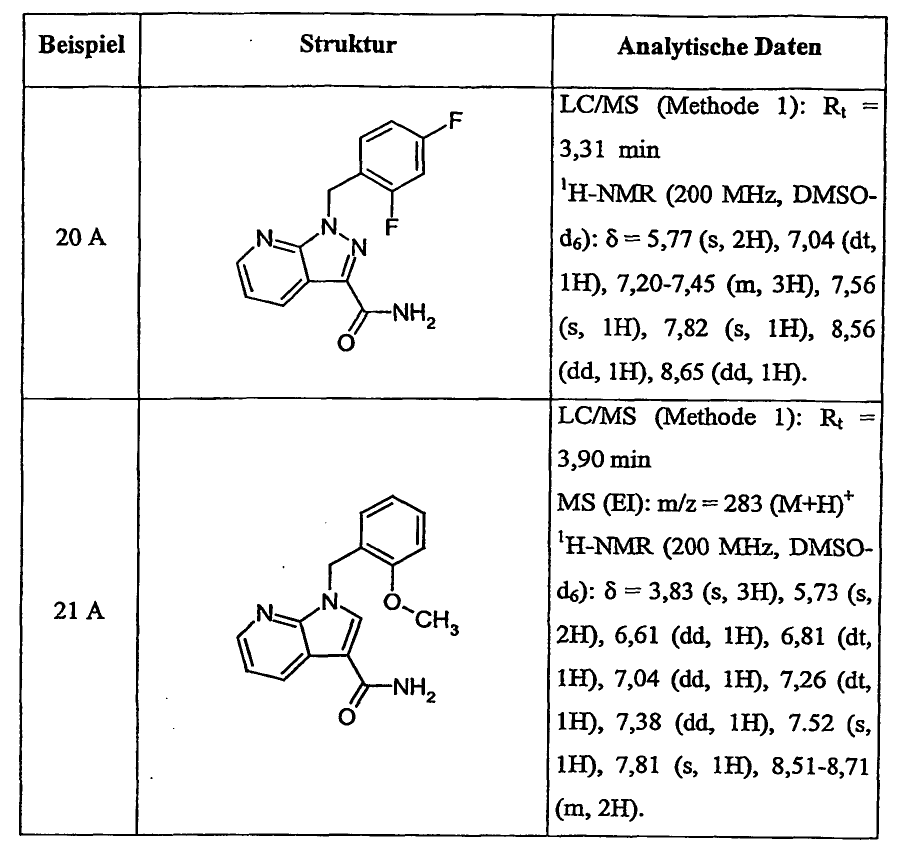

### Beispiel 22 A

### 1-(2-Chlorbenzyl)-1H-pyrazol[3,4-b]pyridin-3-carbonitril

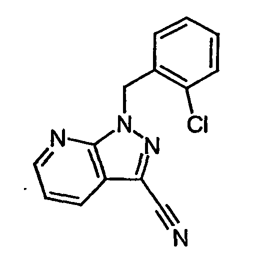

1,19 g (4,16 mmol) 1-(2-Chlorbenzyl)-1H-pyrazol[3,4-b]pyridin-3-carboxamid aus Beispiel 17 A werden in 30 ml THF suspendiert und mit 0,84 g (0,86 ml; 10,66 mmol) Pyridin und 3,00 g (1,75 ml; 10,66 mmol) Trifluoressigsäureanhydrid versetzt. Man rührt über Nacht bei Raumtemperatur. Anschließend wird der Ansatz in 300 ml Wasser gegeben und dreimal mit Ethylacetat extrahiert. Die vereinigten organischen Phasen werden mit gesättigter Natriumhydrogencarbonatlösung und gesättigter Natriumchloridlösung gewaschen, mit Magnesiumsulfat getrocknet und einrotiert.
Gesamtausbeute: 0,880 g (79 % d. Th.)
LC/MS (Methode 1): Rₜ = 4,70 min
MS (EI): m/z = 269 (M+H)⁺
¹H-NMR (200 MHz, DMSO-d₆): δ = 5,92 (s, 2H), 7,18 (dd, 1H), 7,26-7,44 (m, 2H), 7,47-7,61 (m, 2H), 8,52 (dd, 1H), 8,80 (dd, 1H).

### Beispiel 23 A

### 1-(2,3-Difluorbenzyl)-1H-pyrazol[3,4-b]pyridin-3-carbonitril

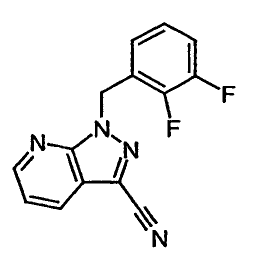

Die Herstellung erfolgt analog zu der in Beispiel 22 A beschriebenen mit 0,84 g (2,91 mmol) 1-(2,3-Difluorbenzyl)-1H-pyrazol[3,4-b]pyridin-3-carboxamid aus Beispiel 18 A, 0,59 g (0,60 ml; 7,46 mmol) Pyridin und 2,10 g (1,22 ml; 7,46 mmol) Trifluoressigsäureanhydrid.
Gesamtausbeute: 0,784 g (99 % d. Th.)
LC/MS (Methode 2): Rₜ = 3,22 min
MS (EI): m/z = 271 (M+H)⁺
¹H-NMR (200 MHz, DMSO-d₆): δ = 5,93 (s, 2H), 7,04-7,28 (m, 2H), 7,33-7,51 (m, 1H), 7,52-7,63 (m, 1H), 8,51 (dd, 1H), 8,81 (dd, 1H).

Die Herstellung der folgenden Verbindungen erfolgt analog zu der in Beispiel 22 A beschriebenen:

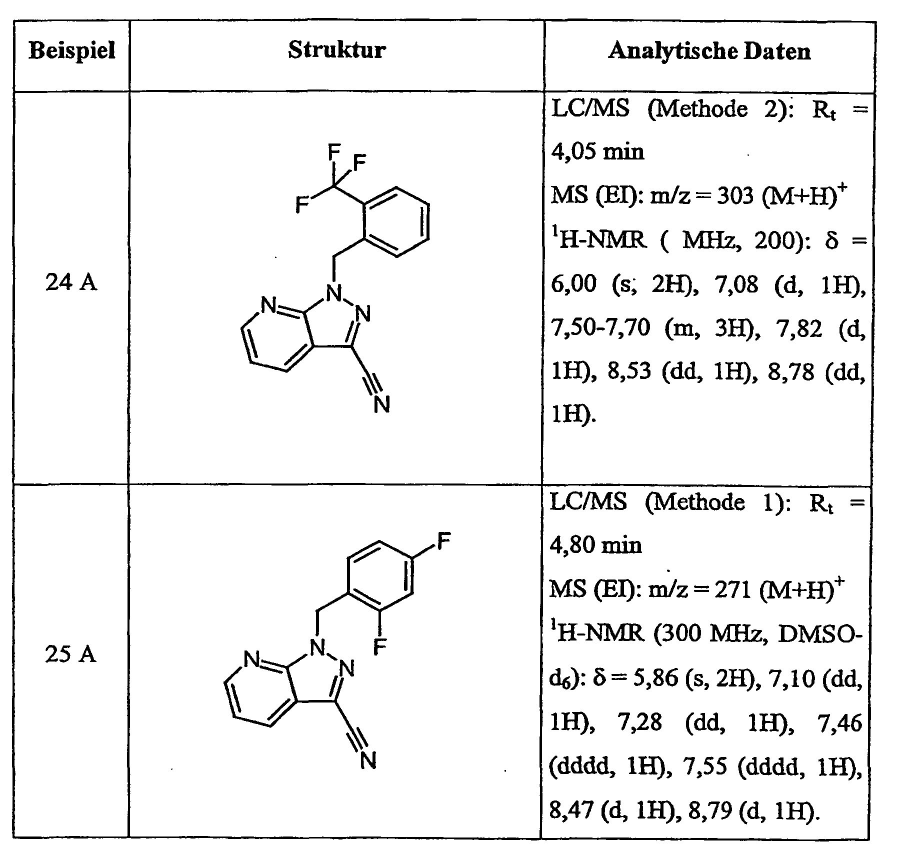

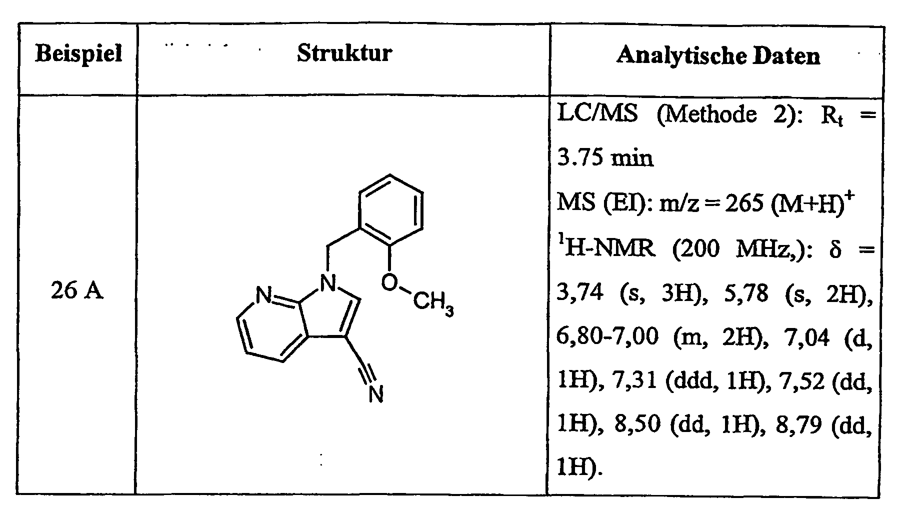

### Beispiel 27 A

### 1-(2-Chlorbenzyl)-1H-pyrazol[3,4-b]pyridin-3-carboximidamid Hydrochlorid

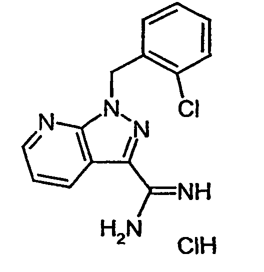

Unter Argon werden 380 mg (1,41 mmol) aus Beispiel 22 A in 6 ml Methanol suspendiert. Dazu gibt man 45,84 mg (0,85 mmol) Natriummethylat und rührt 5 Stunden bei 50°C nach. Anschließend gibt man 189,12 mg (3,54 mmol) Ammoniumchlorid dazu und rührt unter Rückfluss 2 Stunden nach. Die Reaktionslösung wird unter Vakuum einrotiert und der Rückstand mit 25 ml gesättigter Natriumcarbonatlösung suspendiert und mit Ethylacetat dreimal mit je 75 ml extrahiert. Die vereinigten organischen Phasen werden über Magnesiumsulfat getrocknet, filtriert und getrocknet. Der Rückstand wird in 50 ml Diethylether aufgenommen und das Produkt mit 4 normaler Salzsäure in Dioxan gefällt. Der Niederschlag wird filtriert und im Hochvakuum getrocknet.
Gesamtausbeute: 0,200 g (44 % d. Th.)
LC/MS (Methode 2): Rₜ =1,51 min
MS (EI): m/z = 286 (M+H-HCl⁺)
¹H-NMR (300 MHz, DMSO-d₆): δ = 5,95 (s, 2H), 7,07 (dd, 1H), 7,29 (dt, 1H), 7,37 (dt, 1H), 7,49-7,59 (m, 2H), 8,58 (dd, 1H), 8,77 (dd, 1H), 9,42 (br. s, 4H).

### Beispiel 28 A

### 1-(2,3-Difluorbenzyl)-1H-pyrazol[3,4-b]pyridin-3-carboximidamid Hydrochlorid

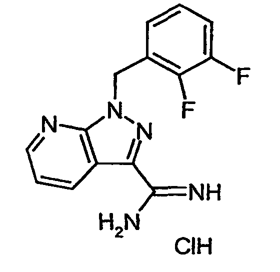

Unter Argon werden 760 mg (2,81 mmol) aus Beispiel 23 A in 10 ml Methanol suspendiert. Dazu gibt man 30,4 mg (0,56 mmol) Natriummethylat und rührt bei RT 4 Stunden nach. Anschließend gibt man 225,6 mg (4,22 mmol) Ammoniumchlorid dazu und rührt bei RT 5 Stunden nach. Nach Zugabe von 20,5 mg konzentrierter Salzsäure wird die Temperatur wieder auf RT abgekühlt und das Produkt im Vakuum vom Lösungsmittel befreit. Der Rückstand wird in 10 %iger Natriumcarbonatlösung suspendiert und mit Ethylacetat dreimal extrahiert. Die vereinigten organischen Phasen werden getrocknet, filtriert und getrocknet. Der Rückstand wird in 15 ml Diethylether aufgenommen und das Produkt mit 1 molarer Salzsäure in Dioxan gefällt. Der Niederschlag wird filtriert und im Hochvakuum getrocknet.
Gesamtausbeute: 0,775 g (76 % d. Th.)
LC/MS (Methode 1): Rₜ = 2,65 min
MS (EI): m/z = 288 (M+H)⁺
¹H-NMR (300 MHz, DMSO-d₆): δ = 5,94 (s, 2H), 7,08-7,24 (m, 1H), 7,34-7,47 (m, 1H), 7,54 (dd, 1H), 8,55 (dd, 1H), 8,78 (dd, 1H), 9,39 (br. s, 4H).

Die Herstellung der folgenden Verbindungen erfolgt analog zu der in Beispiel 27 A beschriebenen:

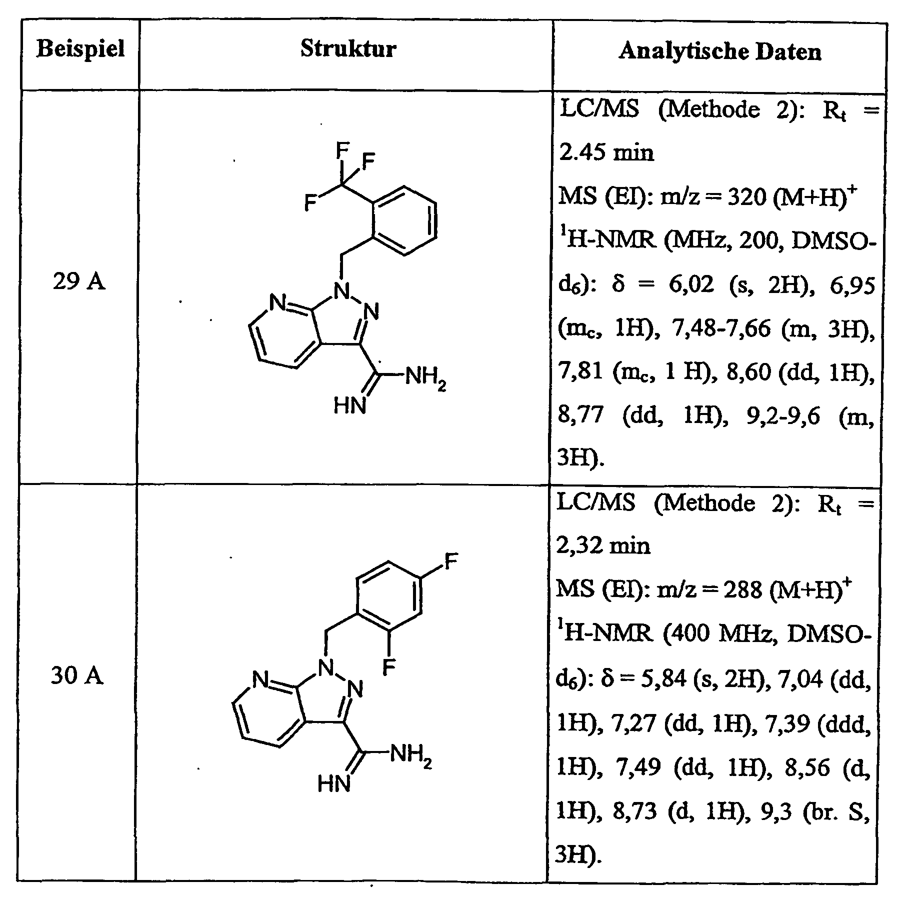

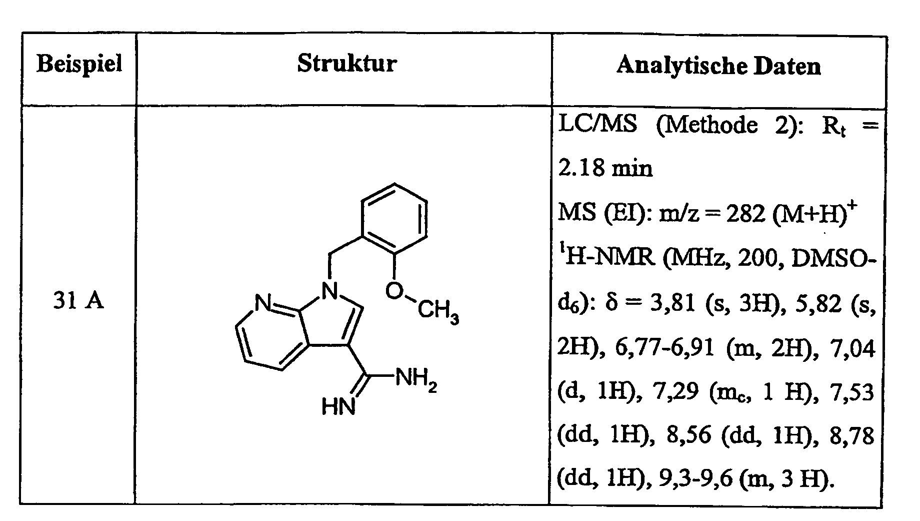

### Beispiel 32 A

### 4-[(Dimethylamino)methylen]-pyridinacetonitril (E/Z-Gemisch)

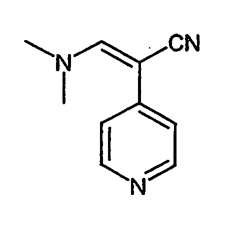

4-Pyridylacetonitril 7,52 g (63,7 mmol) und tert-Butoxybis(dimethylamino)methan 11,09 g (63,7 mmol) werden bei 100°C für 2 h gerührt. Dabei wird frei werdendes Dimethylamin und t-Butanol mittels einer Vakuumpumpe durch leichten Unterdruckstrom zur Atmosphäre abgeführt. Flash-Chromatographie (Dichlormethan/Ethylacetat 50:1 -> 20:1) liefert die Titelverbindung.
Ausbeute: 10,2 g (93 % d.Th.)
R_{f}-Wert: 0,29 (Dichlormethan/EE 20/1)
¹H-NMR (300 MHz, DMSO-d₆): δ = 3,25 (s, 6 H, 2 x CH₃), 7,25 (d, 2 H, Ar-H), 7,80 (s, 1 H, Ar-H), 8,33 (d, 2 H, Ar-H).
MS (ESI pos.): m/z = 174 ([M+H]⁺)

### Beispiel 33 A

### 1-(2-Fluorbenzyl)1H-pyrazolo[3,4-b]pyridin-3-carboxamidin

### 33 A-1 5-Amino-1-(2-fluorbenzyl)-pyrazol-3-carbonsäureethylester

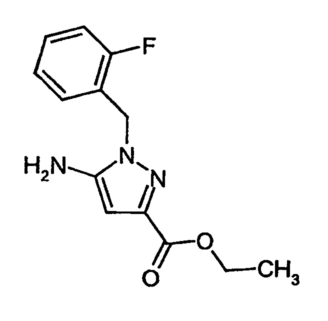

100 g (0,613 mol) Natriumsalz des Cyanobrenztraubensäureethylester (Darstellung analog Borsche und Manteuffel, *Liebigs Ann.* **1934,** *512,* 97) werden unter gutem Rühren unter Argon in 2,5 1 Dioxan bei Raumtemperatur mit 111,75 g (75 ml, 0,98 mol) Trifluoressigsäure versetzt und 10 min gerührt, wobei ein großer Teil des Eduktes in Lösung geht. Dann gibt man 85,93 g (0,613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### 33 A-2 1-(2-Fluorbenzyl)-1H-pyrazolo[3,4-b]pyridin-3-carbonsäureethylester

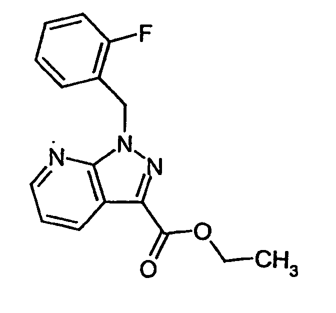

Die aus Beispiel 33 A-1 erhaltene Lösung wird mit 61,25 ml (60,77 g, 0,613 mol) Dimethylaminoacrolein und 56,28 ml (83,88 g, 0,736 mol) Trifluoressigsäure versetzt und unter Argon 3 Tage lang gekocht. Anschließend wird das Lösungsmittel im Vakuum verdampft, der Rückstand in 21 Wasser gegeben und dreimal mit je 11 Ethylacetat extrahiert. Die vereinigten organischen Phasen werden mit Magnesiumsulfat getrocknet und einrotiert. Man chromatographiert auf 2,5 kg Kieselgel und eluiert mit einem Toluol / Toluol-Ethylacetat=4:1 -Gradienten. Ausbeute: 91,6 g (50 % d.Th. über zwei Stufen).
Fp. 85°C
R_{f} (SiO₂, Toluol-Ethylacetat 1:1): 0,83

### 33 A-3 1-(2-Fluorbenzyl)-1H-pyrazolo[3,4-b]pyridin-3-carboxamid

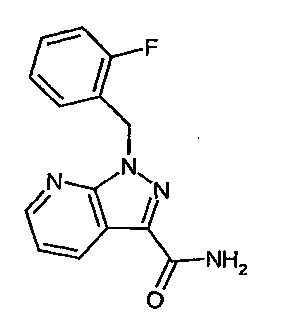

10,18 g (34 mmol) des in Beispiel 33 A-2 erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, Toluol-Ethylacetat 1:1): 0,33

### 33 A-4 3-Cyano-1-(2-fluorbenzyl)-1H-pyrazolo[3,4-b]pyridin

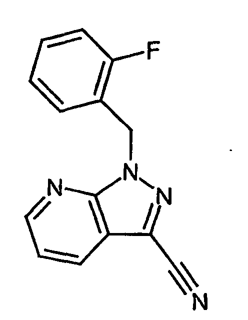

36,1 g (133 mmol) 1-(2-Fluorbenzyl)-1H-pyrazolo[3,4-b]pyridin-3-carboxamid aus Beispiel 33 A-3 werden in 330 ml THF gelöst und mit 27 g (341 mmol) Pyridin versetzt. Anschließend gibt man innerhalb von 10 min 47,76 ml (71,66 g, 341 mmol) Trifluoressigsäureanhydrid hinzu, wobei die Temperatur bis auf 40°C ansteigt. Man rührt über Nacht bei Raumtemperatur. Anschließend wird der Ansatz in 11 Wasser gegeben und dreimal mit je 0,5 1 Ethylacetat extrahiert. Die organische Phase wird mit gesättigter Natriumhydrogencarbonatlösung und mit 1 N Salzsäure gewaschen, mit Magnesiumsulfat getrocknet und einrotiert.
Ausbeute: 33,7 g (100 % d.Th.)
Fp: 81°C
R_{f} (SiO₂, Toluol-Ethylacetat 1:1): 0,74

### 33 A-5 (2-Fluorbenzyl)-1H-pyrazolo[3,4-b]pyridin-3-carboximidsäuremethylester

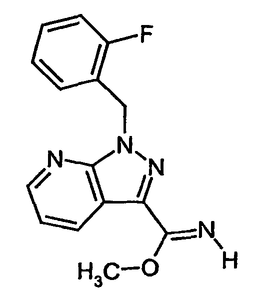

Man löst 30,37 g (562 mmol) Natriummethylat in 1,5 1 Methanol und gibt 36,45 g (144,5 mmol) 3-Cyano-1-(2-fluorbenzyl)-1H-pyrazolo[3,4-b]pyridin (aus Beispiel 33 A-4 hinzu. Man rührt 2 Stunden bei Raumtemperatur und setzt die erhaltene Lösung direkt für die nächste Stufe ein.

### 33 A-6 1-(2-Fluorbenzyl)1H-pyrazolo[3,4-b]pyridin-3-carboximidamid

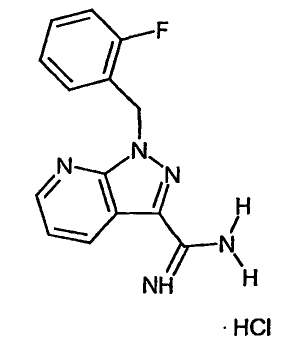

Die aus Beispiel 33 A-5 erhaltene Lösung von (2-Fluorbenzyl)-1H-pyrazolo[3,4-b]pyridin-3-carboximidsäuremethylester in Methanol wird mit 33,76 g (32,19 ml, 562 mmol) Eisessig und 9,28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (DMSO-d₆, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl)

### Beispiel 34 A

### 2-[1-[(2-fluorophenyl)methyl]-1H-pyrazolo[3,4-b]pyridin-3-yl]-5-(4-pyridinyl)-4-pyrimidinamin

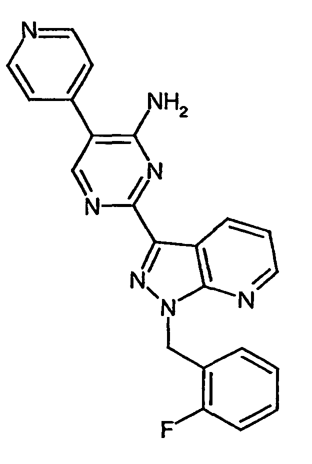

0,50 g (1,9 mmol) 1-(2-Fluorbenzyl)-1H-pyrazolo[3,4-b]pyridin-3-carboximidamid aus Beispiel 33 A und 4-[(Dimethylamino)methylen]-pyridinacetonitril (0,32 g, 1,9 mmol) aus Beispiel 32 A werden in Xylol suspendiert und mit BF₃*OEt₂ (71 µl, 79 mg, 0,56 mmol, 0,3 Äquiv.) versetzt. Nach 19 h bei 140°C lässt man auf Raumtemperatur abkühlen und engt im Vakuum ein. Die Titelverbindung wird durch Flash-Chromatographie an Kieselgel (Dichlormethan:Methanol 20:1) und anschließendes Ausrühren in Acetonitril gereinigt.
Ausbeute: 0,24 g (33 % d. Th.)
R_{f}-Wert: 0,17 (EE/Methanol 20:1)
Fp: 254°C
Retentionzeit: Rₜ = 2.7 min (Säule: Symmetry, C-18, 3,5 µm, 50X2,1. mm, Fluss 0,5 ml/min, 40°C, Gradient: Wasser (+0,1 % Ameisensäure) : Acetonitril (+0,1 % Ameisensäure) bei 0 min: 90:10, bei 7,5 min 10:90))
¹H-NMR (300 MHz, DMSO-d₆): δ = 5,81 (s, 2H, CH₂), 7,0-7,6 (m, 9 H, Ar-H, NH₂), 8,64 (m_{c}, 3 H, Ar-H), 9,05 (d, 1 H, Ar-H)
MS (ESI pos.): m/z = 398 ([M+H]⁺)
MS (ESI neg.): m/z = 396 ([M-H]⁺)

### Beispiel 35 A

### 2-(1H-Pyrazolo[3,4-b]pyridin-3-yl)-5-(4-pyridinyl)-4-pyrimidinamin

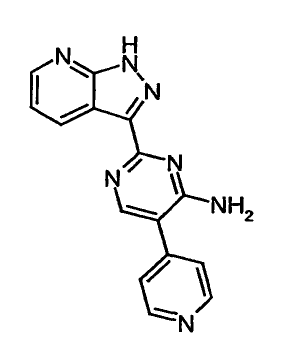

Ca. 15 ml Ammoniak werden in einen mit Trockeneis gekühlten Kolben kondensiert. Dazu gibt man 0,347 g (0,015 mol) Natrium und lässt 30 Minuten nachrühren. Man gibt dann 1,50 g (0,004 mol) der Verbindung aus Beispiel 34 A dazu und lässt 3 Stunden nachrühren. Die Mischung wird mit 1,21 g (0,023 mol) Ammoniumchlorid versetzt und gast über Nacht das restliche Ammoniak über einem Waschturm ab. Zur Aufarbeitung wird mit Wasser versetzt und die Kristalle abgesaugt und getrocknet. Der Rückstand wird säulenchromatographisch (Laufmittel: Dichlormethan:Methanol 8:2) und im Anschluss per RP-HPLC gereinigt
Gesamtausbeute: 0,50 g (65 % d. Th.)
LC/MS (Methode 2): Rₜ =1,09 min
MS (EI): m/z = 290 (M+H)⁺
¹H-NMR (200 MHz, DMSO-d₆): δ = 6,57 (br. s, 2H), 7,25 (dd, 1H), 7,52 (dd, 2H), 7,90 (s, 1H), 8,29 (s, 1H), 8,55 (dd, 1H), 8,70 (dd, 2H), 9,03 (dd, 1H).

### Ausführungsbeispiele

### Beispiel 1

### 2-[1-(2-Chlorbenzyl)-1H-pyrazolo[3,4-b]pyridin-3-yl]-5-(4-pyridinyl)-4-pyrimidinamin

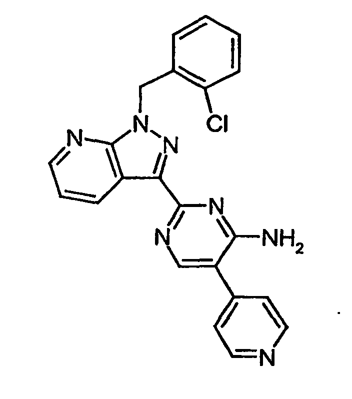

Es werden bei RT 410 mg (1,27 mmol) 1-(2-Chlorbenzyl)-1H-pyrazol[3,4-b]pyridin-3-carboximidamid Hydrochlorid aus Beispiel 27 A und 242,46 mg (1,40 mmol) 4-[(Dimethylamino)methylen]-pyridinacetonitril aus Beispiel 32 A in eine Mischung von 3:1 von Benzylalkohol:Isobutanol suspendiert. Anschließend wird 25,75 mg (0,25 mmol) Triethylamin zugegeben und über Nacht bei 113°C gerührt. Anschließend wird im Vakuum vom Lösungsmittel befreit und auf Kieselgel aufgezogen. Es wird chromatographiert (Laufmittel: Dichlormethan:Methanol 30:1). Die vereinigten sauberen Fraktionen werden wieder vereinigt und mit präparativer RP-HPLC gereinigt.
Gesamtausbeute: 70 mg (13 % d. Th.)
LC/MS (Methode 1): Rₜ = 3,52 min
MS (EI): m/z = 414 (M+H)⁺
¹H-NMR (400 MHz, DMSO-d₆): δ = 5,89 (s, 2H), 6,95 (d, 1H), 7,14 (br. s, 2H), 7,27 (t, 1H), 7,35 (t, 1H), 7,41 (dd, 1H), 7,50-7,58 (m, 3H), 8,28 (s, 1H), 8,61-8,73 (m, 3H), 9,07 (dd, 1H).

### Beispiel 2

### 2-[1-(2,3-Difluorbenzyl)-1H-pyrazolo[3,4-b]pyridin-3-yl]-5-(4-pyridinyl)-4-pyrimidinamin

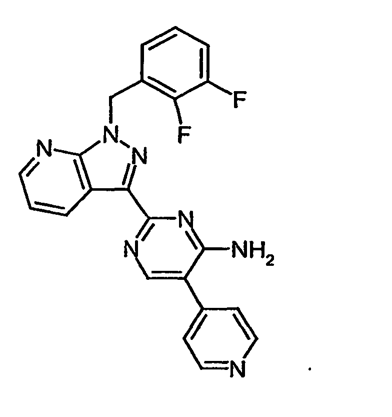

Es werden bei RT 680 mg (1.88 mmol) 1-(2,3-Difluorbenzyl)-1H-pyrazolo[3,4-b]pyridin-3-carboximidamid Hydrochlorid aus Beispiel 28 A und 358 mg (2,07 mmol) 4-[(Dimethylamino)methylen]-pyridinacetonitril aus Beispiel 32 A in eine Mischung von 3:1 von Benzylalkohol:Isobutanol suspendiert. Anschließend werden 38,1 mg (0.38 mmol) Triethylamin zugegeben und über Nacht bei 87-90°C gerührt. Es wird nochmals mit 0,5 eq. 4-[(Dimethylamino)methylen]-pyridinacetonitril aus Beispiel 32 A versetzt und weitere 6 Stunden bei 87-90°C gerührt. Es wird mit 3 ml Benzylalkohol und 19 ml Isobutanol verdünnt und kurz auf 113°C erhitzt. Es wird heiß filtriert und das Filtrat langsam unter Rühren abgekühlt. Anschließend wird im Vakuum vom Lösungsmittel befreit und auf Kieselgel aufgezogen. Es wird chromatographiert (Laufmittel: Dichlormethan:Methanol 30:1-20:1). Die vereinigten sauberen Fraktionen werden wieder vereinigt und mit präparativer RP-HPLC gereinigt.
Gesamtausbeute: 180 mg (23 % d. Th.)
LC/MS (Methode 1): Rₜ = 3,24 min
MS (EI): m/z = 416 (M+H)⁺
¹H-NMR (200 MHz, DMSO-d₆): δ = 5,89 (s, 2H), 6,95-7,08 (m, 1H), 7,09-7,26 (m, 3H), 7,30-7,48 (m, 2H), 7,54 (dd, 2H), 8,28 (s, 1H), 8,61-8,73 (m, 3H), 9,05 (dd, 1H).

Die folgenden Verbindungen werden analog Beispiel 1 hergestellt:

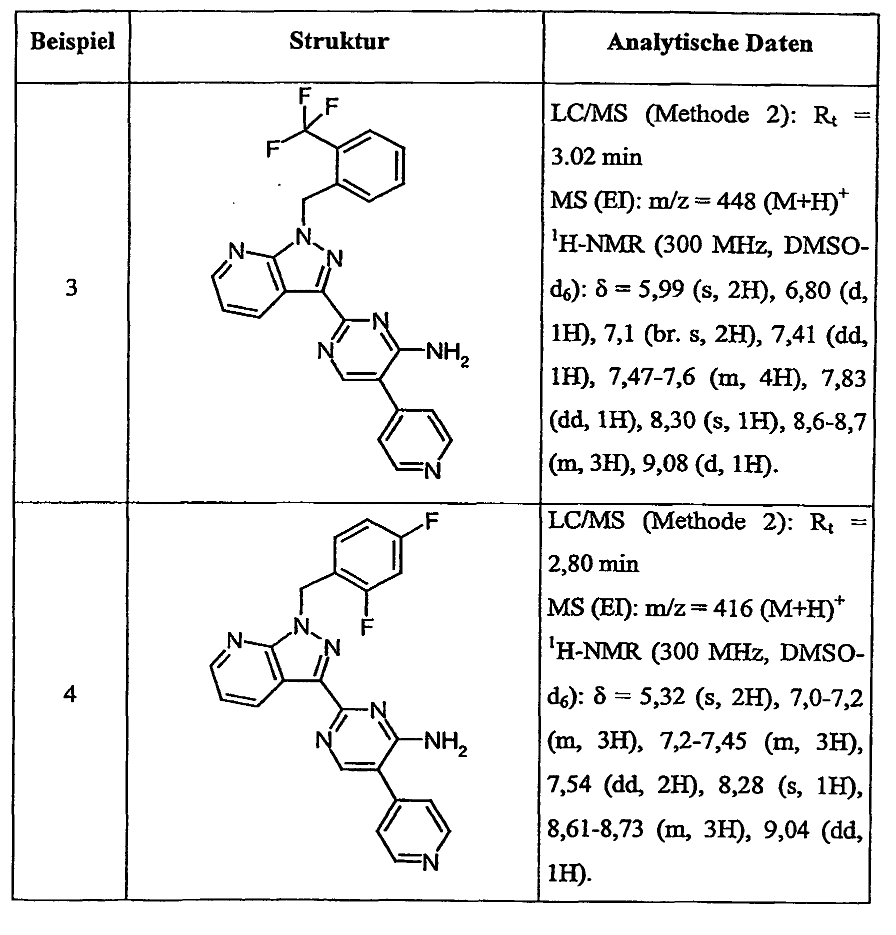

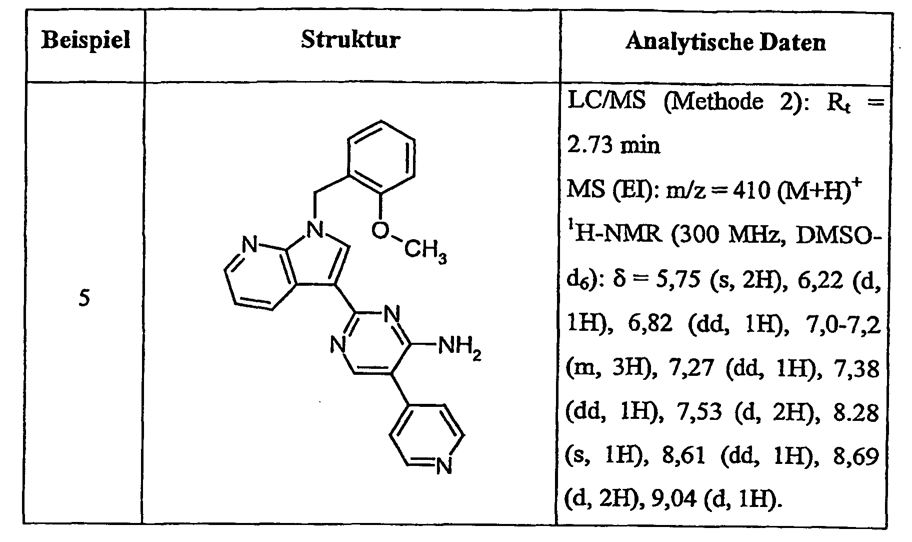

### Beispiel 6

### 2-({3-[4-Amino-5-(4-pyridinyl)-2-pyrimidinyl]-1H-pyrazolo[3,4-b]pyridin-1-yl}-methyl)benzonitril

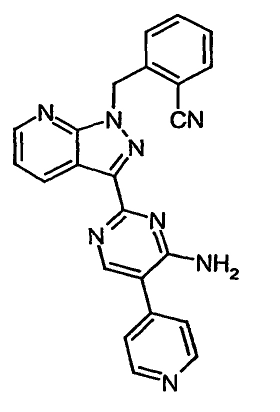

Unter Argon werden 60 mg (0,21 mmol) 2-(1H-Pyrazolo[3,4-b]pyridin-3-yl)-5-(4-pyridinyl)-4-pyrimidinylamin aus Beispiel 35 A in 6 ml Dimethylformamid suspendiert. Nach Zugabe von 26,38 mg (0.25 mmol) Natriumcarbonat wird eine Stunde bei 50°C gerührt. Anschließend gibt man 40,66 mg (0,21 mmol) 2-Cyanobenzylbromid dazu und lässt über Nacht bei 50°C rühren. Zur Aufarbeitung wird filtriert und das Filtrat mit 1 normaler Salzsäure auf pH 4-5 gestellt und mit präparativer RP-HPLC gereinigt.
Gesamtausbeute: 40 mg (48 % d. Th.)
LC/MS (Methode 2): Rₜ= 1,84 min
MS (EI): m/z = 405 (M+H)⁺
¹H-NMR (300 MHz, DMSO-d₆): δ = 5,99 (s, 2H), 6,69 (s, 1H), 7,02-7,17 (m, 2H), 7,22 (d, 1H), 7,41 (dd, 1H), 7,47-7,57 (m, 2H), 7,59-7,68 (m, 1H), 7,85-7,94 (m, 1H), 8,28 (s, 1H), 8,63-8,69 (m, 3H), 9,06 (dd, 1H).

## Patentansprüche

1. Verbindungen der Formel (I) 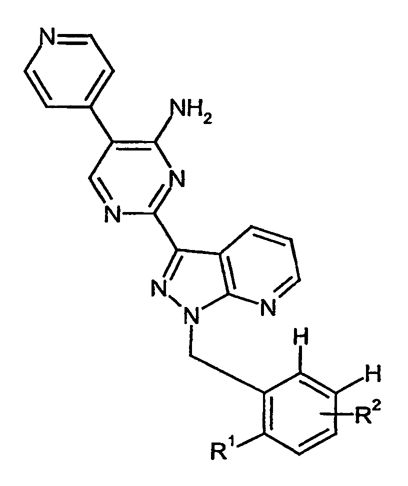 worin
R¹ für Chlor, Cyano, Trifluormethyl oder Methoxy steht
und
R² für Wasserstoff oder Fluor steht
oder
R¹ für Fluor steht und
R² für Fluor steht,
sowie Salze, Isomere und Hydrate davon.

2. Verbindungen der Formel (Ia) nach Anspruch 1 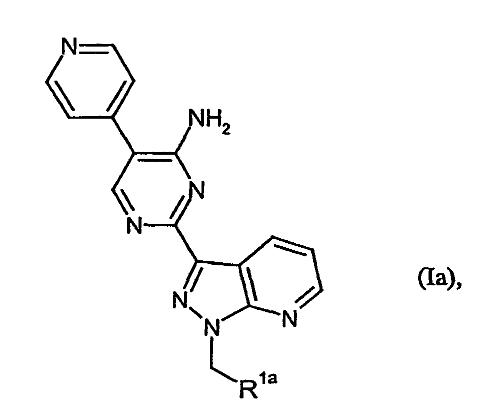 worin
R^{1a} ausgewählt ist aus der Gruppe von 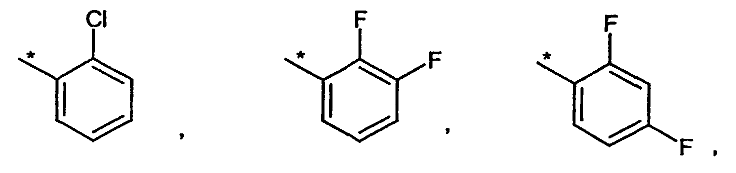 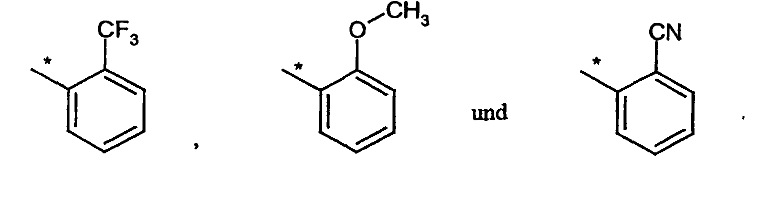
sowie Salze, Isomere und Hydrate davon.

3. Verbindung der Formel (Ia) nach Anspruch 2, bei der
R^{1a} für 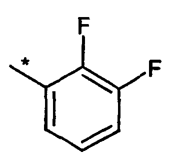 steht,
sowie Salze, Isomere und Hydrate davon.

4. Verbindungen der Formel (I), wie in Anspruch 1 definiert, zur Behandlung von Erkrankungen.

5. Arzneimittel, enthaltend mindestens eine Verbindung der Formel (I), wie in Anspruch 1 definiert, und mindestens einen weiteren Hilfsstoff.

6. Arzneimittel enthaltend mindestens eine Verbindung der Formel (I), wie in Anspruch 1 definiert, in Kombination mit mindestens einem organischen Nitrat oder NO-Donator.

7. Arzneimittel enthaltend mindestens eine Verbindungen der Formel (I), wie in Anspruch 1 definiert, in Kombination mit mindestens einer Verbindung, die den Abbau von cyclischem Guanosinmonophosphat (cGMP) inhibiert.

8. Verwendung von Verbindungen der Formel (I), wie in Anspruch 1 definiert, bei der Herstellung von Arzneimitteln zur Behandlung von Herz-Kreislauf-Erkrankungen.

9. Verwendung von Verbindungen der Formel (I), wie in Anspruch 1 definiert, zur Herstellung von Arzneimitteln zur Behandlung von Hypertonie.

10. Verwendung von Verbindungen der Formel (I), wie in Anspruch 1 definiert, zur Herstellung von Arzneimitteln zur Behandlung von thromboembolischen Erkrankungen und Ischämien.

11. Verwendung von Verbindungen der Formel (I), wie in Anspruch 1 definiert, zur Herstellung von Arzneimitteln zur Behandlung von sexueller Dysfunktion, insbesondere von erektiler Dysfunktion und von weiblicher sexuelle Dysfunktion.

12. Verwendung gemäß einem der Ansprüche 9 bis 11 wobei Verbindungen der Formel (I), wie in Anspruch 1 definiert, in Kombination mit mindestens einem organischen Nitrat oder NO-Donator oder in Kombination mit mindestens einer Verbindung, die den Abbau von cyclischem Guanosinmonophosphat (cGMP) inhibiert, eingesetzt werden.

## Claims

1. Compound of the formula (I) 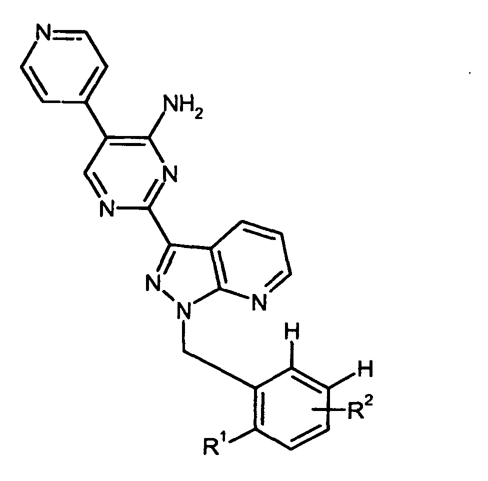 in which
R¹ is chlorine, cyano, trifluoromethyl or methoxy,
and
R² is hydrogen or fluorine,
or
R¹ is fluorine, and
R² is fluorine,
and salts, isomers and hydrates thereof.

2. Compound of the formula (Ia) according to Claim 1 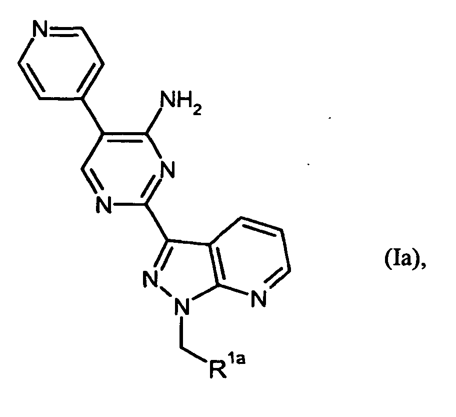 in which
R^{1a} is selected from the group of 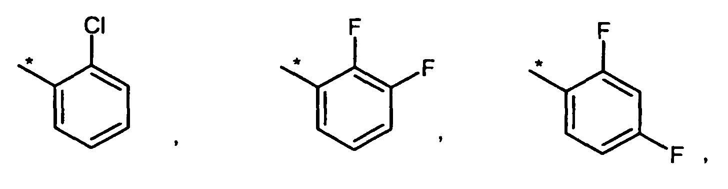 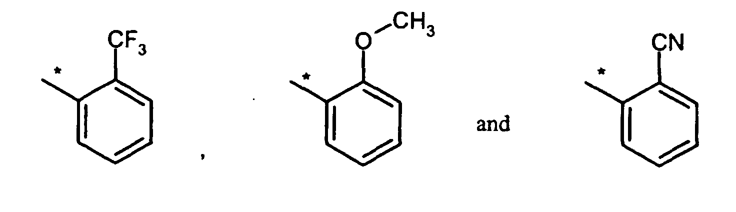
and salts, isomers and hydrates thereof.

3. Compound of the formula (Ia) according to Claim 2, in which
R^{1a} is 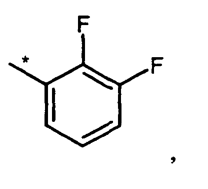 and salts, isomers and hydrates thereof.

4. Compound of the formula (I) according to Claim 1 for the treatment of disorders.

5. Medicament comprising at least one compound of the formula (I) according to Claim 1, and at least one further excipient.

6. Medicament comprising at least one compound of the formula (I) according to Claim 1 in combination with at least one organic nitrate or NO donor.

7. Medicament comprising at least one compound of the formula (I) according to Claim 1 in combination with at least one compound which inhibits the breakdown of cyclic guanosine monophosphate (cGMP).

8. Use of compounds of the formula (I) according to Claim 1 for producing medicaments for the treatment of cardiovascular disorders.

9. Use of compounds of the formula (I) according to Claim 1 for producing medicaments for the treatment of hypertension.

10. Use of compounds of the formula (I) according to Claim 1 for producing medicaments for the treatment of thromboembolic disorders and ischaemias.

11. Use of compounds of the formula (I) according to Claim 1 for producing medicaments for the treatment of sexual dysfunction, especially of erectile dysfunction and of female sexual dysfunction.

12. Use according to any of claims 9 to 11, where compounds of the formula (I) according to Claim 1 are employed in combination with at least one organic nitrate or NO donor or in combination with at least one compound which inhibits the breakdown of cyclic guanosine monophosphate (cGMP).

## Revendications

1. Composés de formule (I) 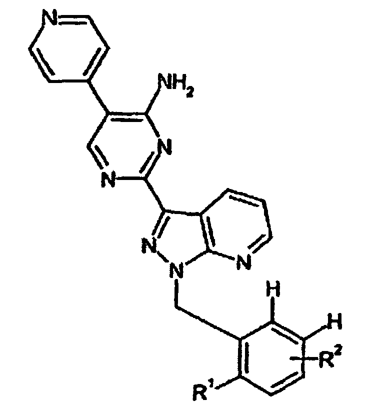 dans laquelle
R¹ représente le chlore, un groupe cyano, trifluorométhyle ou méthoxy
et
R² représente l'hydrogène ou le fluor
ou bien
R¹ représente le fluor et
R² représente le fluor,
ainsi que leurs sels, leurs isomères et leurs hydrates.

2. Composés de formule (Ia) suivant la revendication 1 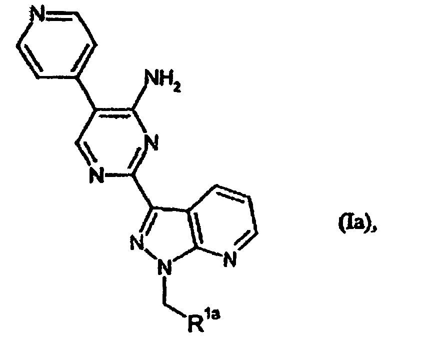 dans laquelle
R^{1a} est choisi dans le groupe formé de 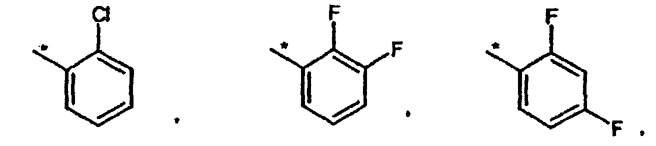 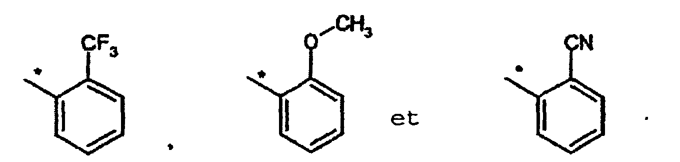
ainsi que leurs sels, leurs isomères et leurs hydrates.

3. Composés de formule (Ia) suivant la revendication 2, formule dans laquelle
R^{1a} représente 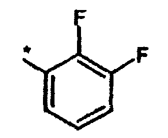
ainsi que ses sels, ses isomères et ses hydrates.

4. Composés de formule (I) tels que définis dans la revendication 1, destinés au traitement de maladies.

5. Médicament, contenant au moins un composé de formule (I), tel que défini dans la revendication 1, et au moins une autre substance auxiliaire.

6. Médicament, contenant au moins un composé de formule (I), tel que défini dans la revendication 1, en combinaison avec au moins un nitrate organique ou un donneur de NO.

7. Médicament, contenant au moins un composé de formule (I), tel que défini dans la revendication 1, en combinaison avec au moins un composé qui inhibe la dégradation du guanosinmonophosphate cyclique (GMPc).

8. Utilisation de composés de formule (I), tels que définis dans la revendication 1, pour la préparation de médicaments destinés au traitement de maladies cardiovasculaires.

9. Utilisation de composés de formule (I), tels que définis dans la revendication 1, pour la préparation de médicaments destinés au traitement de l'hypertonie.

10. Utilisation de composés de formule (I), tels que définis dans la revendication 1, pour la préparation de médicaments destinés au traitement de maladies thromboemboliques et d'ischémies.

11. Utilisation de composés de formule (I), tels que définis dans la revendication 1, pour la préparation de médicaments destinés au traitement d'un dysfonctionnement sexuel, en particulier d'un dysfonctionnement de l'érection et d'un dysfonctionnement sexuel chez la femme.

12. Utilisation suivant l'une des revendications 9 à 11, dans laquelle des composés de formule (I), tels que définis dans la revendication 1, sont utilisés en combinaison avec au moins un nitrate organique ou un donneur de NO ou en combinaison avec au moins un composé qui inhibe la dégradation du guanosinmonophosphate cyclique (GMPc).
